(19) Europäisches Patentamt
European Patent Office
Office européen des brevets

(11) EP 3 590 339 A1

(12) EUROPEAN PATENT APPLICATION
published in accordance with Art. 153(4) EPC

(43) Date of publication:
**08.01.2020 Bulletin 2020/02**

(21) Application number: **18760352.7**

(22) Date of filing: **28.02.2018**

(51) Int Cl.:
***A01N 43/42*** (2006.01) ***A01N 25/10*** (2006.01)
***A01N 25/34*** (2006.01) ***A01P 3/00*** (2006.01)
***C07K 14/78*** (2006.01)

(86) International application number:
**PCT/JP2018/007561**

(87) International publication number:
**WO 2018/159695 (07.09.2018 Gazette 2018/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **28.02.2017 JP 2017037258**

(71) Applicants:
- **Keio University**
  **Tokyo 108-8345 (JP)**
- **Spiber Inc.**
  **Yamagata 997-0052 (JP)**

(72) Inventors:
- **ARAKAWA Kazuharu**
  **Tsuruoka City**
  **Yamagata 997-0035 (JP)**
- **FUJIWARA Masayuki**
  **Tsuruoka City**
  **Yamagata 997-0035 (JP)**
- **KONO Nobuaki**
  **Tsuruoka City**
  **Yamagata 997-0035 (JP)**
- **OHTA Yoshinori**
  **Tsuruoka-shi**
  **Yamagata 997-0052 (JP)**

(74) Representative: **Handley, Matthew Edward et al**
**Venner Shipley LLP**
**200 Aldersgate**
**London EC1A 4HD (GB)**

(54) **MICROBE PROLIFERATION INHIBITOR, METHOD FOR INHIBITING PROLIFERATION OF MICROBES, UV RESISTANCE-IMPROVING AGENT, METHOD FOR IMPROVING UV RESISTANCE, ARTIFICIAL PROTEIN FORMATION BODY AND METHOD FOR MANUFACTURING SAME, ARTIFICIAL PROTEIN SOLUTION, AND COLORING AGENT**

(57) One aspect of the present invention is a microbial growth inhibitor comprising xanthurenic acid.



Printed by Jouve, 75001 PARIS (FR)

## Description

### Technical Field

The present invention relates to a microbial growth inhibitor, a method for inhibiting microbial growth, a UV resistance improver, a method for improving UV resistance, an artificial protein formed product and a method for manufacturing the same, an artificial protein solution, and a coloring agent.

### Background Art

Hereinafter, an effect of inhibiting microbial growth such as bacteria or mold has been imparted to many articles of various types including pharmaceutical products as well as household products and equipment instruments. Such an effect is generally obtained by incorporating a so-called microbial growth inhibitor in various types of articles (e.g., Patent Literature 1). Also, it may be required for articles that are particularly used outdoors to have UV resistance. The UV resistance is achieved, for example, by using a so-called UV resistance improver (e.g., Patent Literature 2).

By the way, in recent years, natural or naturally derived materials have been adopted in various types of fields from the viewpoint of environmental issues, and it is also desired for microbial growth inhibitors or UV resistance improvers to be natural or naturally derived.

### Citation List

### Patent Literature

Patent Literature 1: Japanese Unexamined Patent Publication No. 2002-114946
Patent Literature 2: Japanese Unexamined Patent Publication No. 2016-117862

### Summary of Invention

### Technical Problem

A main object of the present invention is to provide a novel microbial growth inhibitor and UV resistance improver.

### Solution to Problem

The present inventors have found that xanthurenic acid has an effect of inhibiting microbial growth. Specifically, an aspect of the present invention is a microbial growth inhibitor comprising xanthurenic acid. Another aspect of the present invention is a method for inhibiting microbial growth in an article, the method comprising incorporating xanthurenic acid in the article.

The present inventors have also found that xanthurenic acid has an effect of improving UV resistance. Specifically, another aspect of the present invention is a UV resistance improver comprising xanthurenic acid. Another aspect of the present invention is a method for improving UV resistance of an article, the method comprising incorporating xanthurenic acid in the article.

Another aspect of the present invention is an artificial protein formed product comprising an artificial protein and xanthurenic acid. Another aspect of the present invention is a method for manufacturing an artificial protein formed product, comprising: preparing a solution comprising an artificial protein and xanthurenic acid; and performing molding by using the solution. Another aspect of the present invention is an artificial protein solution comprising an artificial protein and xanthurenic acid.

Another aspect of the present invention is a coloring agent comprising xanthurenic acid.

### Advantageous Effects of Invention

According to the present invention, a novel microbial growth inhibitor and UV resistance improver are provided.

### Brief Description of Drawings

Figure 1 is a graph showing measurement results of absorbance (OD600) in Example 1 and Comparative Example 1.
Figure 2 is a graph showing antimicrobial activity values in Examples 2 and 3 and Comparative Examples 2 to 5.

**Description of Embodiments**

Hereinafter, embodiments of the present invention will be described in detail.

The microbial growth inhibitor according to the present embodiment comprises xanthurenic acid. The xanthurenic acid is the compound represented by the following formula (1):

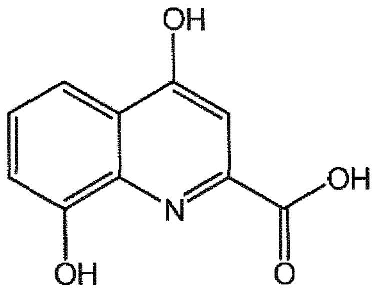

(1)

The microbial growth inhibitor may consist only of xanthurenic acid, or may further comprise other components without inhibiting the microbial growth-inhibiting effect. The method for inhibiting microbial growth in an article according to the present embodiment comprises a step of incorporating xanthurenic acid in the article.

The microbes are not particularly limited and may be, for example, bacteria or may be fungi or the like. The bacteria may be gram-negative bacteria or may be gram-positive bacteria. Examples of the gram-negative bacteria include *Escherichia coli.* Examples of the gram-positive bacteria include *Bacillus subtilis.*

The UV resistance improver according to the present embodiment comprises xanthurenic acid. The UV resistance improver may consist only of xanthurenic acid, or may further comprise other components without inhibiting the UV resistance-improving effect. The method for improving UV resistance of an article according to the present embodiment comprises a step of incorporating xanthurenic acid in the article.

The "articles" in the aforementioned method for inhibiting microbial growth and method for improving UV resistance are common in each other and will therefore be collectively described below.

The article is not particularly limited and may be, for example, an article that is applied to purposes such as pharmaceutical products, medical devices, household products, equipment instruments, electronics, electronic equipment, apparel, bedclothes, ornaments, food products, coloring agents, and parts for various types of vehicles such as automobiles and trains, and comes into contact with human bodies. According to the aforementioned method for inhibiting microbial growth or method for improving UV resistance, pharmaceutical products, medical devices, household products, equipment instruments, electronics, electronic equipment, apparel, bedclothes, ornaments, food products, coloring agent, parts for various types of vehicles such as automobiles and trains, and the like comprising xanthurenic acid are obtained.

One example of the article includes an article comprising a protein. The protein is not particularly limited and can be, for example, a structural protein. The structural protein means a protein that forms or retains a structure, a form, or the like *in vivo.* Such a structural protein can be, for example, fibroin, keratin, collagen, elastin, or resilin. These proteins are used, each alone or in an appropriate combination, as the structural protein. The structural protein is preferably fibroin.

The fibroin may be at least one member selected from the group consisting of, for example, silk fibroin, spider silk fibroin, and hornet silk fibroin. Particularly, the structural protein may be silk fibroin, spider silk fibroin or a combination thereof. In the case of using silk fibroin and spider silk fibroin in combination, the ratio of the silk fibroin is, for example, 40 parts by mass or less, 30 parts by mass or less, or 10 parts by mass or less, with respect to 100 parts by mass of the spider silk fibroin.

Silk yarn is fiber (cocoon filaments) obtained from a cocoon produced by a silkworm larva of *Bombyx mori.* In general, one cocoon filament is constituted by two threads of silk fibroin and a colloid (sericin) which covers them from the outside. The silk fibroin is constituted by a large number of fibrils. The silk fibroin is covered with four layers of sericin. For practical use, a silk filament obtained by dissolving and removing the outside sericin by smelting is used for apparel purposes. General silk yarn has a specific gravity of 1.33, a fineness of 3.3 decitex on average, and a fiber length on the order of 1300 to 1500 m. The silk fibroin is obtained with a natural or domestic silkworm cocoon or used or discarded silk cloth as a raw material.

The silk fibroin may be sericin-removed silk fibroin, sericin-unremoved silk fibroin, or a combination thereof. The sericin-removed silk fibroin is the one purified after removing sericin covering silk fibroin, and other fats and the like.

The silk fibroin thus purified is preferably used as a freeze-dried powder. The sericin-unremoved silk fibroin is unpurified silk fibroin from which sericin or the like is not removed.

The spider silk fibroin may contain a spider silk polypeptide selected from the group consisting of a natural spider silk protein and a polypeptide derived from a natural spider silk protein.

Examples of the natural spider silk protein include major dragline silk proteins, weft proteins, and minor ampullate silk proteins. The major dragline silk has repeat regions consisting of a crystalline region and a non-crystalline region (also referred to as an amorphous region) and therefore possesses both high stress and stretching properties. The weft of spider silk has a feature of lacking a crystalline region and having repeat regions consisting of a non-crystalline region. The weft is inferior in stress to the major dragline silk, but has high stretching properties.

The major dragline silk protein is produced in the major ampullate of a spider and has a feature of being excellent in toughness. Examples of the major dragline silk protein include major ampullate spidroins MaSp1 and MaSp2 derived from *Nephila clavipes,* and ADF3 and ADF4 derived from *Araneus diadematus.* ADF3 is one of the two primary dragline silk proteins of *Araneus diadematus.* The polypeptide derived from the natural spider silk protein may be a polypeptide derived from the dragline silk protein. The polypeptide derived from ADF3 is relatively easy to synthesize and has excellent characteristics in terms of strong elongation and toughness.

The weft protein is produced in the flagelliform gland of a spider. Examples of the weft protein include flagelliform silk proteins derived from *Nephila clavipes.*

The polypeptide derived from the natural spider silk protein can be a recombinant spider silk protein. Examples of the recombinant spider silk protein include variants, analogs and derivatives of natural spider silk proteins. One preferable example of such a polypeptide is a recombinant spider silk protein of a major dragline silk protein (also referred to as a "polypeptide derived from a major dragline silk protein").

Examples of the major dragline silk-derived protein or the silkworm silk-derived protein, which is a fibroin-like protein, include a protein comprising the domain sequence represented by formula 1: [(A)n motif-REP]m or formula 2: [(A)n motif-REP]m-(A)n motif. In this context, (A)n motif represents an amino acid sequence composed mainly of alanine residues, and n is 2 to 27. n can be an integer of 2 to 20, 4 to 27, 4 to 20, 8 to 20, 10 to 20, 4 to 16, 8 to 16, or 10 to 16. The ratio of the number of alanine residues to the total number of amino acid residues in (A)n motif can be 40% or more and may be 60% or more, 70% or more, 80% or more, 83% or more, 85% or more, 86% or more, 90% or more, 95% or more, or 100% (which means being constituted only by alanine residues). At least seven of a plurality of (A)n motifs present in the domain sequence may be constituted only by alanine residues. REP represents an amino acid sequence constituted by 2 to 200 amino acid residues. REP may be an amino acid sequence constituted by 10 to 200 amino acid residues. m represents an integer of 2 to 300 and may be an integer of 10 to 300. A plurality of (A)n motifs present may be the same amino acid sequences with each other or may be different amino acid sequences. A plurality of REP present may be the same amino acid sequences with each other or may be different amino acid sequences. Specific examples thereof include a protein comprising the amino acid sequence represented by SEQ ID NO:1.

Examples of the protein derived from the weft protein include a protein comprising the domain sequence represented by formula 3: [REP2]o (wherein REP2 represents an amino acid sequence constituted by Gly-Pro-Gly-Gly-X wherein X represents one amino acid selected from the group consisting of alanine (Ala), serine (Ser), tyrosine (Tyr) and valine (Val); and o represents an integer of 8 to 300). Specific examples thereof include a protein comprising the amino acid sequence represented by SEQ ID NO: 2. The amino acid sequence represented by SEQ ID NO: 2 is the one in which an amino acid sequence (referred to as a PR1 sequence) from the 1220th residue to the 1659th residue counted from the N terminus, which corresponds to a repeat moiety and a motif, of a partial sequence (NCBI Accession No: AAF36090, GI: 7106224) of the flagelliform silk protein of *Nephila clavipes* obtained from the NCBI database, is bound with a C-terminal amino acid sequence from the 816th residue to the 907th residue counted from the C terminus of a partial sequence (NCBI Accession No: AAC38847, GI: 2833649) of the flagelliform silk protein of *Nephila clavipes* obtained from the NCBI database, and the amino acid sequence represented by SEQ ID NO: 6 (tag sequence and hinge sequence) is added to the N terminus of the bound sequences.

Examples of the collagen-derived protein include a protein comprising the domain sequence represented by formula 4: [REP3]p (wherein p represents an integer of 5 to 300; REP3 represents an amino acid sequence constituted by Gly-X-Y wherein X and Y represent an arbitrary amino acid residue other than Gly; and a plurality of REP3 present may be the same amino acid sequences with each other or may be different amino acid sequences). Specific examples thereof include a protein comprising the amino acid sequence represented by SEQ ID NO: 3. The amino acid sequence represented by SEQ ID NO: 3 is the one in which the amino acid sequence represented by SEQ ID NO: 6 (tag sequence and hinge sequence) is added to the N terminus of an amino acid sequence from the 301st residue to the 540th residue, which corresponds to a repeat moiety and a motif, of a partial sequence (NCBI GenBank Accession No: CAA56335.1, GI: 3702452) of human collagen type 4 obtained from the NCBI database.

Examples of the resilin-derived protein include a protein comprising the domain sequence represented by formula 5: [REP4]q (wherein q represents an integer of 4 to 300; REP4 represents an amino acid sequence constituted by Ser-J-J-Tyr-Gly-U-Pro wherein J represents an arbitrary amino acid residue, and it is particularly preferable to be an

amino acid residue selected from the group consisting of Asp, Ser and Thr; and U represents an arbitrary amino acid residue, and it is particularly preferable to be an amino acid residue selected from the group consisting of Pro, Ala, Thr and Ser; and a plurality of REP4 present may be the same amino acid sequences with each other or may be different amino acid sequences). Specific examples thereof include a protein comprising the amino acid sequence represented by SEQ ID NO: 4. The amino acid sequence represented by SEQ ID NO: 4 is the one in which the amino acid sequence represented by SEQ ID NO: 6 (tag sequence and hinge sequence) is added to the N terminus of an amino acid sequence from the 19th residue to the 321st residue of a sequence prepared by substituting Thr at the 87th residue by Ser and substituting Asn at the 95th residue by Asp in the amino acid sequence of resilin (NCBI GenBank Accession No. NP 611157, GI: 24654243).

Examples of the elastin-derived protein include a protein having the amino acid sequence of NCBI GenBank Accession No. AAC98395 (human), 147076 (sheep), NP786966 (bovine), or the like. Specific examples thereof include a protein comprising the amino acid sequence represented by SEQ ID NO: 5. The amino acid sequence represented by SEQ ID NO: 5 is the one in which the amino acid sequence represented by SEQ ID NO: 6 (tag sequence and hinge sequence) is added to the N terminus of an amino acid sequence from the 121st residue to the 390th residue of the amino acid sequence of NCBI GenBank Accession No. AAC98395.

The protein contained as a main component in the protein raw material fiber can be produced, for example, by expressing the nucleic acid of interest by a host transformed with an expression vector having a nucleic acid sequence encoding the protein, and one or more regulatory sequences operably linked to the nucleic acid sequence.

A method for manufacturing a gene encoding the protein contained as a main component in the protein raw material fiber is not particularly limited. The gene can be manufactured, for example, by a method of utilizing, amplifying and cloning a gene encoding a natural structural protein by polymerase chain reaction (PCR) or the like, or chemical synthesis. The method for chemically synthesizing the gene is not particularly limited, and the gene can be chemically synthesized by, for example, a method of linking, by PCR or the like, oligonucleotides automatically synthesized in AKTA oligopilot plus 10/100 (GE Healthcare Japan Corp.) or the like on the basis of amino acid sequence information on the structural protein obtained from the Web database of NCBI or the like. In this respect, in order to facilitate the purification or confirmation of the protein, a gene encoding a protein consisting of an amino acid sequence in which a start codon and an amino acid sequence consisting of His10 tag are added to the N terminus of the amino acid sequence described above, may be synthesized.

The regulatory sequence is a sequence that regulates the expression of a recombinant protein in a host (e.g., a promoter, an enhancer, a ribosomal binding sequence, and a transcription termination sequence), and can be appropriately selected according to the type of the host. An inducible promoter that functions in host cells and is capable of inducing the expression of the protein of interest may be used as the promoter. The inducible promoter is a promoter that can control transcription by a physical factor such as the presence of an inducing substance (expression inducer), the absence of a repressor molecule, or elevation or reduction in temperature, osmotic pressure or pH value.

The type of the expression vector can be appropriately selected according to the type of the host from a plasmid vector, a virus vector, a cosmid vector, a fosmid vector, an artificial chromosome vector, and the like. The one that is capable of replicating autonomously in host cells or capable of being integrated into the chromosome of the host, and contains a promoter at a position where a nucleic acid encoding the protein of interest can be transcribed is preferably used as the expression vector.

Any of prokaryotes, and eukaryotes such as yeasts, filamentous fungi, insect cells, animal cells and plant cells can be preferably used as the host.

Preferable examples of the prokaryote include bacteria belonging to the genus *Escherichia,* the genus *Brevibacillus,* the genus *Serratia,* the genus *Bacillus,* the genus *Microbacterium,* the genus *Brevibacterium,* the genus *Corynebacterium* and the genus *Pseudomonas.* Examples of the microbe belonging to the genus *Escherichia* include *Escherichia coli.* Examples of the microbe belonging to the genus *Brevibacillus* include *Brevibacillus agri.* Examples of the microbe belonging to the genus *Serratia* include *Serratia liquefaciens.* Examples of the microbe belonging to the genus *Bacillus* include *Bacillus subtilis.* Examples of the microbe belonging to the genus *Microbacterium* include *Microbacterium ammoniaphilum.* Examples of the microbe belonging to the genus *Brevibacterium* include *Brevibacterium divaricatum.* Examples of the microbe belonging to the genus *Corynebacterium* include *Corynebacterium ammoniagenes.* Examples of the microbe belonging to the genus *Pseudomonas* include *Pseudomonas putida.*

Examples of the vector to which the nucleic acid encoding the protein contained as a main component in the protein raw material fiber is introduced include pBTrp2 (manufactured by Boehringer Mannheim GmbH), pGEX (manufactured by Pharmacia), pUC18, pBluescript II, pSupex, pET22b, pCold, pUB110, and pNCO2 (Japanese Unexamined Patent Publication No. 2002-238569).

Examples of the eukaryotic host include yeasts and filamentous fungi (mold, etc.). Examples of the yeast include yeasts belonging to the genus *Saccharomyces,* the genus *Pichia,* and the genus *Schizosaccharomyces.* Examples of the filamentous fungus include filamentous fungi belonging to the genus *Aspergillus,* the genus *Penicillium,* and the genus *Trichoderma.*

Examples of the vector include YEP13 (ATCC37115) and YEp24 (ATCC37051). Any method of introducing DNA to the host cells can be used as a method for introducing the expression vector to the host cells. Examples thereof include a method using calcium ions [Proc. Natl. Acad. Sci. USA, 69, 2110 (1972)], an electroporation method, a spheroplast method, a protoplast method, a lithium acetate method, and a competent method.

Direct expression as well as secretory production, fusion protein expression, or the like in accordance with a method described in Molecular Cloning, 2nd edition, etc. can be performed as a method for expressing the nucleic acid by the host transformed with the expression vector.

The protein can be manufactured, for example, by culturing the host transformed with the expression vector in a culture medium, and producing and accumulating the protein into the culture medium, followed by collection from the culture medium. A method for culturing the host in a culture medium can be performed according to a method that is usually used in the culture of the host.

In the case where the host is a prokaryote such as *Escherichia coli* or an eukaryote such as a yeast, any of natural media and synthetic media may be used as the culture medium as long as the medium contains a carbon source, a nitrogen source and inorganic salts, etc. utilizable by the host and the culture of the host is efficiently performed.

The carbon source can be the one utilizable by the transformed microbe, and, for example, glucose, fructose, sucrose, and molasses containing them, hydrocarbons such as starch and starch hydrolysates, organic acids such as acetic acid and propionic acid, and alcohols such as ethanol and propanol can be used. For example, ammonia, ammonium salts of inorganic acids or organic acids such as ammonium chloride, ammonium sulfate, ammonium acetate and ammonium phosphate, other nitrogen-containing compounds, and peptone, meat extracts, yeast extracts, corn steep liquor, casein hydrolysates, soymeal and soymeal hydrolysates, various types of fermented bacterial cells and digests thereof can be used as the nitrogen source. For example, monobasic potassium phosphate, dibasic potassium phosphate, magnesium phosphate, magnesium sulfate, sodium chloride, ferrous sulfate, manganese sulfate, copper sulfate and calcium carbonate can be used as the inorganic salt.

The culture of the prokaryote such as *Escherichia coli* or the eukaryote such as a yeast can be performed, for example, under aerobic conditions such as shake culture or submerged aeration-agitation culture. The culture temperature is, for example, 15 to 40°C. The culture time is usually 16 hours to 7 days. It is preferable that the pH of the culture medium during culture should be kept at 3.0 to 9.0. The adjustment of the pH of the culture medium can be performed by using an inorganic acid, an organic acid, an alkali solution, urea, calcium carbonate and ammonia, etc.

Antibiotics such as ampicillin and tetracycline may be added, if necessary, to the culture medium during culture. When the microbe transformed with the expression vector using an inducible promoter as the promoter is cultured, an inducer may be added, if necessary, to the medium. When the microbe transformed with the expression vector using, for example, lac promoter is cultured, isopropyl-p-D-thiogalactopyranoside or the like may be added to the medium; and when the microbe transformed with the expression vector using trp promoter is cultured, indoleacrylic acid or the like may be added thereto.

The isolation and purification of the protein can be performed by a method that is usually used. For example, in the case where the protein is expressed in a dissolved state in cells, the host cells are recovered by centrifugation after the completion of culture and suspended in an aqueous buffer solution, and then, the host cells are disrupted with an ultrasonic disrupting machine, a French press, a Manton Gaulin homogenizer and Dynomill, etc. to obtain cell-free extracts. From a supernatant obtained by centrifuging the cell-free extracts, a purified preparation can be obtained by using methods that are usually used in protein isolation and purification, i.e., methods such as a solvent extraction method, a salting out method with ammonium sulfate or the like, a desalting method, a precipitation method with an organic solvent, an anion-exchange chromatography method using a resin such as diethylaminoethyl (DEAE)-Sepharose or DIAION HPA-75 (manufactured by Mitsubishi Kasei Kogyo K.K.), a cation-exchange chromatography method using a resin such as S-Sepharose FF (manufactured by Pharmacia), a hydrophobic chromatography method using a resin such as butyl Sepharose or phenyl Sepharose, a gel filtration method using a molecular sieve, an affinity chromatography method, a chromatofocusing method, and an electrophoresis method such as isoelectric focusing, alone or in combination.

In the case where the protein is expressed by forming an insoluble form in cells, the host cells are similarly recovered, then disrupted, and centrifuged to thereby recover the insoluble form of the protein as a precipitated fraction. The recovered insoluble form of the protein can be solubilized with a protein denaturant. After the operation, a purified preparation of the protein can be obtained by the same isolation and purification method as described above. In the case where the protein is extracellularly secreted, the protein can be recovered from a culture supernatant. Specifically, a culture supernatant is obtained by treating the cultures by an approach such as centrifugation, and from the culture supernatant, a purified preparation can be obtained by using the same isolation and purification method as described above.

The protein raw material fiber containing the protein as described above as a main component is manufactured by a publicly known approach. Specifically, in manufacturing protein raw material fiber comprising, for example, spider silk fibroin, spider silk fibroin manufactured by using a host or the like transformed with the expression vector is first

added, together with an inorganic salt as a dissolution promoter, to a solvent such as dimethyl sulfoxide (DMSO), N,N-dimethylformamide (DMF), hexafluoroisopropanol (HFIP), or formic acid, and dissolved to prepare a dope solution. Subsequently, this dope solution can be used and spun by a publicly known spinning approach such as wet spinning, dry spinning, or dry-wet spinning to obtain a protein raw material.

The shape of the article is not limited and can be, for example, fiber, a film, a molded body, a gel, a porous body, or a particle.

The "steps of incorporating xanthurenic acid in the article" in the aforementioned method for inhibiting microbial growth and method for improving UV resistance are common in each other and will therefore be collectively described below.

In this step, xanthurenic acid is incorporated in the article. In the present specification, "incorporating xanthurenic acid in the article" means that the article and xanthurenic acid are allowed to coexist with each other such that the desired effect (microbial growth-inhibiting effect or UV resistance-improving effect) is obtained, and encompasses, for example, allowing the article and xanthurenic acid to be mixed with each other, and allowing xanthurenic acid to be present on the surface or in the inside of the article.

In one embodiment, in this step, xanthurenic acid can be contained in the article (the article and the xanthurenic acid can be mixed with each other, for example, in a liquid state or a powder and granular state) by mixing the article and the xanthurenic acid with each other. In another embodiment, in this step, xanthurenic acid can be contained in the article (the xanthurenic acid can be present on the surface of the article) by imparting the xanthurenic acid (for example, applying a solution containing the xanthurenic acid) onto the surface of the article. In another embodiment, in this step, xanthurenic acid can be contained in the article (the xanthurenic acid can be present in the inside of the article) by adding the xanthurenic acid into a raw material for manufacturing the article.

In this step, it is preferable to allow xanthurenic acid to be present in the inside of the article. Hereinafter, specific examples of a method for incorporating xanthurenic acid to be present in the inside of the article will be described in more detail on an article shape basis by taking the case where the article comprises a protein as an example.

In the case where the article is fiber comprising a protein, the protein is first dissolved in a predetermined solvent while xanthurenic acid is dissolved or dispersed to prepare a dope solution. The solvent is not particularly limited as long as the solvent is capable of dissolving the protein, and can be, for example, dimethyl sulfoxide (DMSO), N,N-dimethylformamide (DMF), or hexafluoroisopropanol (HFIP).

The dope solution may further contain an inorganic salt, if necessary. Examples of the inorganic salt include inorganic salts consisting of a Lewis acid and a Lewis base. Examples of the Lewis base include oxoacid ions (nitric acid ions, perchloric acid ions, etc.), metal oxoacid ions (permanganic acid ions, etc.), halide ions, thiocyanic acid ions, and cyanic acid ions. Examples of the Lewis acid include metal ions such as alkali metal ions and alkaline earth metal ions, polyatomic ions such as ammonium ions, and complex ions. Specific examples of the inorganic salt consisting of the Lewis acid and the Lewis base include: lithium salts such as lithium chloride, lithium bromide, lithium iodide, lithium nitrate, lithium perchlorate, and lithium thiocyanate; calcium salts such as calcium chloride, calcium bromide, calcium iodide, calcium nitrate, calcium perchlorate, and calcium thiocyanate; iron salts such as iron chloride, iron bromide, iron iodide, iron nitrate, iron perchlorate, and iron thiocyanate; aluminum salts such as aluminum chloride, aluminum bromide, aluminum iodide, aluminum nitrate, aluminum perchlorate, and aluminum thiocyanate; potassium salts such as potassium chloride, potassium bromide, potassium iodide, potassium nitrate, potassium perchlorate, and potassium thiocyanate; sodium salts such as sodium chloride, sodium bromide, sodium iodide, sodium nitrate, sodium perchlorate, and sodium thiocyanate; zinc salts such as zinc chloride, zinc bromide, zinc iodide, zinc nitrate, zinc perchlorate, and zinc thiocyanate; magnesium salts such as magnesium chloride, magnesium bromide, magnesium iodide, magnesium nitrate, magnesium perchlorate, and magnesium thiocyanate; barium salts such as barium chloride, barium bromide, barium iodide, barium nitrate, barium perchlorate, and barium thiocyanate; and strontium salts such as strontium chloride, strontium bromide, strontium iodide, strontium nitrate, strontium perchlorate, and strontium thiocyanate.

The viscosity of the dope solution can be appropriately set according to a spinning method and can be, for example, 100 to 15000 cP (centipoise) at 35°C. The viscosity of the dope solution can be measured by using, for example, trade name "EMS viscometer" manufactured by Kyoto Electronics Manufacturing Co., Ltd.

This dope solution is used and spun by a publicly known spinning approach such as wet spinning, dry spinning, or dry-wet spinning. Preferable examples of the spinning method include wet spinning.

In the wet spinning, the dope solution is extruded into a coagulation solution (coagulation solution bath) from a spinneret (nozzle), and the protein can be solidified in the coagulation solution to thereby obtain undrawn yarn having a thread shape. The coagulation solution can be a solution that can be desolvated, and examples thereof include lower alcohols having 1 to 5 carbon atoms, such as methanol, ethanol and 2-propanol, and acetone. Water may be appropriately added to the coagulation solution. It is preferable that the temperature of the coagulation solution should be 0 to 30°C. In the case of using a syringe pump having a nozzle of 0.1 to 0.6 mm in diameter as the spinneret, the extrusion rate is preferably 0.2 to 6.0 ml/hr, more preferably 1.4 to 4.0 ml/hr, per hole. The length of the coagulation solution bath can be a length at which desolvation is efficiently performed, and is, for example, 200 to 500 mm. The take-up speed of the

undrawn yarn can be, for example, 1 to 20 m/min, and it is preferable to be 1 to 3 m/min. The residence time can be, for example, 0.01 to 3 minutes, and it is preferable to be 0.05 to 0.15 minutes. Alternatively, drawing (pre-drawing) may be performed in the coagulation solution. In order to suppress the evaporation of a lower alcohol, the coagulation solution may be maintained at a low temperature and taken up in the state of undrawn yarn. The coagulation solution bath may be established in multiple stages, and drawing may be performed in each stage or a particular stage, if necessary.

The undrawn yarn (or pre-drawn yarn) obtained by the method described above can be prepared into drawn yarn (fibroin fiber) through a drawing step. Examples of the drawing method include moist heat drawing and dry heat drawing.

The moist heat drawing can be performed in hot water, in a solution of an organic solvent or the like added to hot water, or in steam heating. The temperature can be, for example, 50 to 90°C, and 75 to 85°C is preferable. In the moist heat drawing, the undrawn yarn (or pre-drawn yarn) can be drawn, for example, by 1 to 10 times, and it is preferable to be drawn by 2 to 8 times.

The dry heat drawing can be performed by using an electric tube furnace, a dry hot plate, or the like. The temperature can be, for example, 140°C to 270°C, and 160°C to 230°C is preferable. In the dry heat drawing, the undrawn yarn (or pre-drawn yarn) can be drawn, for example, by 0.5 to 8 times, and it is preferable to be drawn by 1 to 4 times.

The moist heat drawing and the dry heat drawing may be performed each alone, or they may be performed in multiple stages or in combination. Specifically, the moist heat drawing and the dry heat drawing can be performed in an appropriate combination, in such a way that the first-stage drawing is performed by the moist heat drawing, and the second-stage drawing is performed by the dry heat drawing, or the first-stage drawing is performed by the moist heat drawing, the second-stage drawing is performed by the moist heat drawing, and further, the third-stage drawing is performed by the dry heat drawing.

The final draw ratio in the drawing step is, for example, 5 to 20 times with respect to the undrawn yarn (or pre-drawn yarn), and it is preferable to be 6 to 11 times.

The protein, after the drawing into fiber, may be chemically cross-linked between the intrafiber polypeptide molecules of the protein. Examples of the functional groups that can be cross-linked include an amino group, a carboxyl group, a thiol group and a hydroxy group. For example, the amino group of a lysine side chain contained in the polypeptide can be cross-linked through an amide bond to the carboxyl group of a glutamic acid or aspartic acid side chain by dehydration condensation. The cross-linking may be caused by performing dehydration condensation reaction under vacuum heating, or the cross-linking may be caused with a dehydrating condensing agent such as carbodiimide.

The cross-linking between the polypeptide molecules may be performed by using a cross-linking agent such as carbodiimide or glutaraldehyde, or may be performed by using an enzyme such as transglutaminase. The carbodiimide is a compound represented by the formula $R^1NC=NR^2$ (wherein $R^1$ and $R^2$ each independently represent an organic group including an alkyl group having 1 to 6 carbon atoms, and a cycloalkyl group). Specific examples of the carbodiimide include 1-ethyl-3-(3-dimethylaminopropyl)carbodiimide hydrochloride (EDC), N,N'-dicyclohexylcarbodiimide (DCC), 1-cyclohexyl-3-(2-morpholinoethyl)carbodiimide, and diisopropylcarbodiimide (DIC). Among them, EDC and DIC are preferable because the ability to form an amide bond between the polypeptide molecules is high and cross-linking reaction is easy to perform.

It is preferable for the cross-linking treatment to impart a cross-linking agent to the protein fiber and perform cross-linking by vacuum heating and drying. The cross-linking agent may be imparted as a pure product to the fibroin fiber, or the one diluted into a concentration of 0.005 to 10% by mass with a lower alcohol having 1 to 5 carbon atoms and a buffer solution, etc. may be imparted to the fibroin fiber. It is preferable that the cross-linking treatment should be performed at a temperature of 20 to 45°C for 3 to 42 hours. By the cross-linking treatment, higher stress (strength) can be imparted to the fibroin fiber.

The fiber thus obtained can be applied as fiber or yarn to a woven fabric, a knit fabric, a braided article, a nonwoven fabric, and the like, and can also be applied to high-strength purposes such as ropes, surgical sutures, flexible stoppers for electric parts, and physiologically active materials for transplantation (e.g., artificial ligaments and aortic bands).

In the case where the article is a film, the protein is first dissolved in a predetermined solvent while xanthurenic acid is dissolved or dispersed to prepare a dope solution, as in the case where the article is fiber.

Then, the dope solution is cast-molded onto base material surface, and drying and/or desolvation is performed. Specifically, the dope solution is first applied to base material surface with a predetermined thickness (e.g., to be 1 to 1000 μm in terms of a thickness after drying and/or desolvation).

The base material can be a resin substrate, a glass substrate, a metal substrate, or the like. The base material is preferably a resin substrate from the viewpoint that a film after cast molding can be easily peeled. The resin substrate can be, for example, a polyethylene terephthalate (PET) film, a fluorine resin film such as polytetrafluoroethylene, a polypropylene (PP) film, or a release film in which a silicone compound is immobilized on the surface of such a film. The base material is more preferably a PET film or a release film in which a silicone compound is immobilized on PET film surface, from the viewpoint that it is stable against a DMSO solvent, the dope solution can be stably cast-molded, and

a film after molding can be easily peeled.

The drying and/or the desolvation is performed by at least one approach selected from, for example, vacuum drying, hot air drying, drying in air, and dipping in a liquid. The dipping in a liquid may be performed in water or in an alcohol solution such as a lower alcohol having 1 to 5 carbon atoms, such as methanol, ethanol, or 2-propanol, or the cast film may be dipped in a mixed solution of water and an alcohol or the like and desolvated. The temperature of the desolvation solution (coagulation solution) is preferably 0 to 90°C. It is preferable that the solvent should be eliminated as much as possible. In the case of drawing the film in a solution, the desolvation may be performed at the same time with the drawing. Alternatively, the desolvation may be performed after drawing the film.

The undrawn film after the drying and/or the desolvation can be uniaxially drawn or biaxially drawn in water. The biaxial drawing may be sequential drawing or may be simultaneous biaxial drawing. Multistage drawing of two or more stages may be performed. The draw ratio is preferably 1.01 to 6 times, more preferably 1.05 to 4 times, both longitudinally and transversely. Within this range, stress-strain balance is easy to bring. It is preferable that the drawing in water should be performed at a water temperature of 20 to 90°C. It is preferable that the film after the drawing should be heat-fixed for 5 to 600 seconds by dry heat of 50 to 200°C. By this heat fixation, dimensional stability at ordinary temperature is obtained. The uniaxially drawn film becomes a uniaxially oriented film, and the biaxially drawn film becomes a biaxially oriented film.

The film may be a color film. In this case, a coloring agent such as a dye is dissolved or dispersed in, for example, a DMSO solvent to prepare a DMSO coloring solution, and a solution obtained by mixing this coloring solution with a dope solution cast-molded in the same way as mentioned above to thereby prepare a film. Then, an undrawn colored film is prepared by drying and/or desolvation, or a drawn film is prepared by drawing. The color film can be applied to a reflector, a marker, an anti-UV film, slitted yarn, and the like.

In the case where the article is a molded body, a composition comprising a protein and xanthurenic acid is introduced to a mold of a pressure molding machine, and then, the mold is heated while the composition is pressurized. The heating and the pressurization are continued until the composition reaches a predetermined temperature under a predetermined pressure, to obtain a heated and pressurized composition. Subsequently, the temperature of the mold is lowered by using a cooling machine (e.g., a spot cooler), and when the composition reaches a predetermined temperature, the contents are taken out to obtain a molded body.

It is preferable that the heating should be performed at 80 to 300°C, 100 to 180°C is more preferable, and 100 to 130°C is further preferable. It is preferable that the pressurization should be performed at 5 kN or higher, 10 kN or higher is more preferable, and 20 kN or higher is further preferable. After reaching predetermined heating and pressurization conditions, the time for which treatment under the conditions is continued (incubation conditions) is preferably 0 to 100 minutes, more preferably 1 to 50 minutes, further preferably 5 to 30 minutes.

In the case where the article is a gel, a protein is first dissolved in a predetermined solvent while xanthurenic acid is dissolved or dispersed to prepare a protein solution. The solvent can be, for example, a dimethyl sulfoxide solvent, a solvent of an inorganic salt added to dimethyl sulfoxide, or a solvent of an inorganic salt added to N,N-dimethylformamide. The solvent may further contain an alcohol and/or water in addition to such a solvent.

Subsequently, the solvent in the protein solution is replaced with a water-soluble solvent. The water-soluble solvent refers to a solvent containing water, and examples thereof include water, water-soluble buffer solutions, and physiological saline. The water-soluble solvent is preferably water from the viewpoint that adaptability to human bodies is high. The water is not particularly limited, and pure water, distilled water, ultrapure water, or the like can be used.

It is preferable that the step of performing replacement with the water-soluble solvent should be performed by a method of placing the protein solution in a dialysis membrane, which is then dipped in the water-soluble solvent, and exchanging the water-soluble solvent one or more times. Specifically, the protein solution is placed in a dialysis membrane, which is then left standing for 3 hours in the water-soluble solvent in 100 or more times the amount of the solution (for one run), and it is more preferable to repeat this exchange of the water-soluble solvent a total of three or more times. The dialysis membrane can be the one impermeable to the protein and can be, for example, a cellulose dialysis membrane. By repeating the replacement of the water-soluble solvent, the amount of the solvent present in the protein solution can be reduced to close to zero. In the latter half of the step of performing replacement with the water-soluble solvent, the dialysis membrane may not be used.

A step of pouring the protein into a mold form to form a predetermined shape may be established between the step of preparing the protein solution and the step of replacing the solvent with the water-soluble solvent, or a step of cutting the gel to form a predetermined shape may be established after the step of replacing the solvent with the water-soluble solvent.

In this way, for example, a gel of the protein having a water content of 85.0 to 99.9% by mass can be obtained. The gel having such a water content is useful in artificial cartilage or artificial skin of an organism, a wound healing agent, a drug entrapment carrier for drug delivery systems, a buffering material, and the like.

In the case where the article is a porous body, a drying step of drying the gel obtained by the aforementioned method is further established. In the drying step, pores are formed by eliminated water, and a porous body having, for

example, continuous holes, is obtained.

In the drying step, it is preferable to use vacuum freeze drying. The degree of vacuum at the time of vacuum freeze drying is preferably 200 Pa or less, more preferably 150 Pa or less, further preferably 100 Pa or less. By the vacuum drying, water is evaporated from the protein gel, and the temperature is decreased by the latent heat of this evaporation to create a frozen state. The temperature of the polypeptide at the time of vacuum freeze drying is preferably 70°C or lower, more preferably 60°C or lower, further preferably 50°C or lower. Preliminary freezing may be performed at a temperature of -10 to -45°C for approximately 10 to 36 hours prior to the vacuum freeze drying. The water content of the porous body after the freeze drying is preferably 5.0% by mass or less, more preferably 3.0% by mass or less.

In the case where the article is a particle, a protein is first dissolved in predetermined solvent while xanthurenic acid is dissolved or dispersed to prepare a protein solution, as in the case where the article is a gel. Subsequently, a step of replacing the solvent in the protein solution with a water-soluble solvent to thereby obtain an aqueous solution of the protein, and a step of drying the aqueous solution of the protein are further established.

The step of replacing the protein solution with a water-soluble solvent to thereby obtain an aqueous solution of the protein can be the same as the step of replacing the solvent in the protein solution with a water-soluble solvent in the case where the article is a gel. The step of drying the aqueous solution of the protein can be the same as the drying step in the case where the article is a porous body.

In the step of incorporating xanthurenic acid in the article as described above, the lower limit of the content of the xanthurenic acid with respect to the total mass of the article is preferably 0.02% by mass or more, more preferably 0.1% by mass or more, further preferably 0.2% by mass or more, particularly preferably 0.4% by mass or more. On the other hand, the upper limit of the content is not particularly limited, but is preferably 2.0% by mass or less. The content may be 0.02 to 2.0% by mass, 0.1 to 2.0% by mass, 0.2 to 2.0% by mass, or 0.4 to 2.0% by mass.

As described above, in the present embodiment, by incorporating xanthurenic acid in the article, microbial growth in the article can be inhibited, and UV resistance of the article can be improved.

In one embodiment, an artificial protein formed product that can inhibit microbial growth and can improve UV resistance is provided by utilizing such characteristics of xanthurenic acid. Specifically, the artificial protein formed product according to one embodiment comprises an artificial protein and xanthurenic acid. The "artificial protein" means, for example, a protein manufactured in a microbe or the like by a gene recombination technique, or a protein manufactured by chemical synthesis.

The artificial protein can be the aforementioned structural protein, and a preferable form of the structural protein is the same as mentioned above. The artificial protein formed product can be, for example, fiber, a film, a formed product, a gel, a porous body, or a particle.

The lower limit of the content of the xanthurenic acid in the artificial protein formed product is preferably 0.02% by mass or more, more preferably 0.1% by mass or more, further preferably 0.2% by mass or more, particularly preferably 0.4% by mass or more, with respect to the total mass of the artificial protein formed product. On the other hand, the upper limit of the content is not particularly limited, but is preferably 2.0% by mass or less with respect to the total mass of the artificial protein formed product. The content may be 0.02 to 2.0% by mass, 0.1 to 2.0% by mass, 0.2 to 2.0% by mass, or 0.4 to 2.0% by mass, with respect to the total mass of the artificial protein formed product.

A method for manufacturing an artificial protein formed product such as fiber, a film, a formed product, a gel, a porous body, or a particle may be the same as mentioned above about each. Specifically, in one embodiment (in the case of incorporating xanthurenic acid to be present in the inside of the artificial protein formed product), the method for manufacturing an artificial protein formed product comprises: a step of providing a solution containing an artificial protein and xanthurenic acid (artificial protein solution); and a step of performing formation by using the solution.

In the step of providing a solution, the lower limit of the content of the xanthurenic acid in the solution is preferably 0.02% by mass or more, more preferably 0.1% by mass or more, further preferably 0.2% by mass or more, particularly preferably 0.4% by mass or more, with respect to the total mass of the solution. On the other hand, the upper limit of the content is not particularly limited, but is preferably 2.0% by mass or less with respect to the total mass of the solution. The content may be 0.02 to 2.0% by mass, 0.1 to 2.0% by mass, 0.2 to 2.0% by mass, or 0.4 to 2.0% by mass, with respect to the total mass of the solution.

The artificial protein formed product described above is preferably used for various purposes mentioned above and particularly preferably used for purposes that should inhibit microbial growth and purposes that require UV resistance among them.

## Examples

Hereinafter, the present invention will be described further specifically with reference to Examples; however, the present invention is not limited by the following Examples.

<Microbial growth-inhibiting effect>

(Example 1)

An *Escherichia coli* wild strain (W3110) was cultured in M9 medium (7 mg/ml $K_2HPO_4$, 3 mg/ml $KH_2PO_4$, 0.5 mg/ml NaCl, 1 mg/ml $NH_4Cl$, 1 mg/ml glucose, 1 mM $MgSO_4 \cdot 7H_2O$, 14.7 μg/ml $CaCl_2$) containing 10 μg/ml thiamine. In preparing the medium, a Tris-HCl buffer solution (pH 8) was used instead of water in consideration of the solubility of xanthurenic acid. The obtained overnight cultures were diluted into 0.8% with 20 ml of a fresh medium containing 1 mM xanthurenic acid (Wako Pure Chemical Industries, Ltd.) to obtain a sample solution. Absorbance (OD600) was measured as to this sample solution every hour by using Nano Photometer (Implen GmbH).

(Comparative Example 1)

The preparation and measurement of a sample solution were performed in the same way as in Example 1 except that the overnight cultures were diluted with a fresh medium containing no xanthurenic acid.

The measurement results of absorbance (OD600) in Example 1 and Comparative Example 1 are shown in Figure 1. From Figure 1, it is evident that in Example 1 compared with Comparative Example 1, the absorbance (OD600) was smaller; thus the proliferation of *Escherichia coli* was inhibited.

(Example 2)

1. Manufacture of spider silk protein (spider silk fibroin: PRT410)

(Synthesis of gene encoding spider silk protein, and construction of expression vector)

Engineered fibroin (hereinafter, also referred to as "PRT410") having the amino acid sequence represented by SEQ ID NO: 1 was designed on the basis of the nucleotide sequence and amino acid sequence of *Nephila clavipes*-derived fibroin (GenBank Accession No: P46804.1, GI: 1174415).

The amino acid sequence represented by SEQ ID NO: 1 has an amino acid sequence in which the amino acid sequence of *Nephila clavipes*-derived fibroin was subjected to the substitution, insertion and deletion of amino acid residues for the purpose of improvement in productivity, and the amino acid sequence represented by SEQ ID NO: 6 (tag sequence and hinge sequence) added to the N terminus thereof.

A nucleic acid encoding the designed PRT410 was synthesized. For the nucleic acid, an NdeI site and an EcoRI site were added to the 5' end and to downstream of a stop codon, respectively. The nucleic acid was cloned into a cloning vector (pUC118). Then, this nucleic acid was excised by restriction enzyme treatment with NdeI and EcoRI and then recombined with protein expression vector pET-22b(+) to obtain an expression vector.

*Escherichia coli* BLR (DE3) was transformed with the obtained pET22b(+) expression vector. The transformed *Escherichia coli* was cultured for 15 hours in 2 mL of LB medium containing ampicillin. The culture solution was added to 100 mL of a medium for seed culture (Table 1) such that $OD_{600}$ became 0.005. The culture solution temperature was kept at 30°C, and flask culture was performed for approximately 15 hours until $OD_{600}$ became 5, to obtain a seed culture solution.

[Table 1]

| Reagent | Concentration (g/L) |
|---|---|
| Glucose | 5.0 |
| $KH_2PO_4$ | 4.0 |
| $K_2HPO_4$ | 9.3 |
| Yeast Extract | 6.0 |
| Ampicillin | 0.1 |

The seed culture solution was added to a jar fermenter supplemented with 500 ml of a production medium (Table 2) such that $OD_{600}$ became 0.05. The culture solution temperature was kept at 37°C, and culture was performed by constantly controlling pH to 6.9. Also, the dissolved oxygen concentration in the culture solution was maintained at 20% of the dissolved oxygen saturation concentration.

[Table 2]

| Reagent | Concentration (g/L) |
|---|---|
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| ADEKANOL (ADEKA Corp., LG-295S) | 0.1 (mL/L) |

Immediately after glucose in the production medium was completely consumed, a feed solution (455 g of glucose/1 L, 120 g of Yeast Extract/1 L) was added at a rate of 1 mL/min. The culture solution temperature was kept at 37°C, and culture was performed by constantly controlling pH to 6.9. The culture was performed for 20 hours while the dissolved oxygen concentration in the culture solution was maintained at 20% of the dissolved oxygen saturation concentration. Then, 1 M isopropyl-p-thiogalactopyranoside (IPTG) was added to the culture solution such that the final concentration became 1 mM, to induce the expression of PRT799. At the point in time when 20 hours passed after the IPTG addition, the culture solution was centrifuged to recover bacterial cells. SDS-PAGE was performed by using bacterial cells prepared from the culture solutions before the IPTG addition and after the IPTG addition, and the expression of PRT799 was confirmed from the appearance of a band of a size corresponding to PRT799 dependent on the IPTG addition.

(Purification of spider silk fibroin)

The bacterial cells recovered after 2 hours from the addition of IPTG were washed with 20 mM Tris-HCl buffer (pH 7.4). The bacterial cells after the washing were suspended in 20 mM Tris-HCl buffer solution (pH 7.4) containing approximately 1 mM PMSF, and the cells were disrupted in a high-pressure homogenizer (GEA Niro Soavi). The disrupted cells were centrifuged to obtain precipitates. The obtained precipitates were washed with 20 mM Tris-HCl buffer solution (pH 7.4) until becoming a high purity. The precipitates after the washing were suspended in 8 M guanidine buffer solution (8 M guanidine hydrochloride, 10 mM sodium dihydrogen phosphate, 20 mM NaCl, 1 mM Tris-HCl, pH 7.0) to become a concentration of 100 mg/mL, and dissolved by stirring with a stirrer at 60°C for 30 minutes. After the dissolution, dialysis was performed against water by using a dialysis tube (cellulose tube 36/32 manufactured by Sanko Junyaku Co., Ltd.). A white aggregated protein (PRT799) obtained after the dialysis was recovered by centrifugation. Water was removed from the recovered aggregated protein in a freeze drying machine to obtain a freeze-dried powder of PRT799.

2. Manufacture of spider silk protein film

A necessary amount of xanthurenic acid (Tokyo Chemical Industry Co., Ltd.: CAS 59-00-7) to be added was dissolved in 0.1 N sodium hydroxide to prepare a xanthurenic acid solution. The spider silk protein powder (PRT410) obtained as described above was added to 30% aqueous ethanol solution to become a solution of approximately 9 w/v%, and a protein solution was obtained by stirring in a glass vial.

The obtained xanthurenic acid solution and protein solution were mixed in a glass vial such that the content of the xanthurenic acid was 1.21 w/w% with respect to the content of the protein. Subsequently, the glass vial was hermetically sealed and heated to 95°C to dissolve the protein, and then, the glass vial was opened, and the solution was left at room temperature for 2 days in a state developed on a Petri dish, and thereby dried. The obtained film was dipped in 70% aqueous methanol solution for 30 minutes or longer. After the dipping, the aqueous methanol solution was discarded, and the film was dried.

*Escherichia coli* BLR was inoculated to 5 ml of LB medium containing 20 μg/ml kanamycin and cultured overnight at 37°C. Subsequently, after confirming that the bacterial count was approximately $1 \times 10^9$ by using CDA-1000 (Sysmex Corp.), a culture solution was obtained by dilution such that the bacterial count (viable bacterial count immediately after inoculation) was approximately $1 \times 10^5$.

Subsequently, the measurement of an antimicrobial activity value was performed with reference to JIS Z 2801. Specifically, a 100 mg aliquot of a film (sample film) was first dipped in 500 μl of the culture solution in a Petri dish. A PET film was placed on the sample film such that the culture solution was dried off, and the Petri dish was hermetically sealed with a parafilm, followed by culture at 37°C for 24 hours. The sample film and the culture solution were transferred

to a 50 ml tube, and the culture solution remaining in the Petri dish was washed by adding 20 ml of a dilution solution (Sysmex Corp., CELLPACK) to the Petri dish, and after the solution was transferred to the 50 ml tube, the tube was preserved on ice. Subsequently, the sample was further diluted 200-fold, and the bacterial count (viable bacterial count after culture) was measured by using CDA-1000 (Sysmex Corp.). From the measurement results, the antimicrobial activity value was calculated according to the following expression:

Antimicrobial activity value = {log(Viable bacterial count after culture of a control sample) - log(Viable bacterial count immediately after inoculation of the control sample)} - {log(Viable bacterial count after culture of the test sample) - log(Viable bacterial count immediately after inoculation of the test sample)}

A film prepared in the same way as in Example 1 except that the xanthurenic acid solution was not used was used as the control sample.

(Comparative Examples 2 and 3)

The preparation of a film and the measurement of an antimicrobial activity value were performed in the same way as in Example 1 except that a solution in which each of Lanaset (Tanakanao Senryoten K.K.) in Comparative Example 2 and Deluxe (Tanakanao Senryoten K.K.) in Comparative Example 3 was dissolved instead of xanthurenic acid in 30% aqueous ethanol solution was used.

(Example 3)

The preparation of a film and the measurement of an antimicrobial activity value were performed in the same way as in Example 1 except that LB medium containing no kanamycin was used instead of LB medium containing kanamycin at the time of culture of *Escherichia coli.* In this case, the culture solution also contained bacteria other than *Escherichia coli.*

(Comparative Examples 4 and 5)

The preparation of a film and the measurement of an antimicrobial activity value were performed in the same way as in Example 3 except that a solution in which each of Lanaset (Tanakanao Senryoten K.K.) in Comparative Example 4 and Deluxe (Tanakanao Senryoten K.K.) in Comparative Example 5 was dissolved instead of xanthurenic acid in 30% aqueous ethanol solution was used.

The antimicrobial activity values of Example 2 and Comparative Examples 2 and 3 are shown in Figure 2(a), and the antimicrobial activity values of Example 3 and Comparative Examples 4 and 5 are shown in Figure 2(b). From Figure 2, it is evident that the films comprising xanthurenic acid (Examples 2 and 3), compared with the films comprising no xanthurenic acid (Comparative Examples 2 to 5), had a large antimicrobial activity value and exhibited a proliferation-inhibiting effect on *Escherichia coli* or the other bacteria.

<UV resistance-improving effect>

(Example 4)

[Manufacture of protein fiber]

(Preparation of dope solution)

To dimethyl sulfoxide (DMSO), the aforementioned spider silk fibroin (PRT410) was added to become a concentration of 24% by mass, then LiCl at a concentration of 4.0% by mass was added as a dissolution promoter, further a dye at a concentration of 0.2% by mass and zinc acetate dihydrate at a concentration of 0.22% by mass were added,

and dissolved for 20 hours by using a stirrer. Then, foreign matter and bubbles were removed to prepare a dope solution. The solution viscosity of the dope solution was 7000 cP (centipoise) at 90°C.

(Spinning)

The dope solution prepared as described above was charged into a syringe and discharged into a coagulation bath of 100% by mass of methanol by using a gear pump from a mono-hole nozzle of 0.2 mm in diameter. The amount discharged was adjusted to 41 to 67 mg/min. After the coagulation, washing and drawing were performed in a washing bath of 100% by mass of methanol. After the washing and the drawing, drying was performed by using a hot roller of 60°C, and the obtained fiber was taken up.

[UV irradiation conditions]

The taken-up fiber was irradiated with UV under conditions of a temperature of 63°C, a humidity of 65% rh, an illuminance of 115 $mW/cm^2$, and 0.5 to 17 hours by using super promotion lightfastness testing equipment "Eye Super UV Tester SUV-W161" manufactured by Iwasaki Electric Co., Ltd.

[Fiber strength measurement conditions]

The obtained fiber was fixed to a test paper piece at a gripping distance of 20 mm by using an adhesive, and stress, elongation, and toughness measurement was performed at a tensile rate of 10 cm/min under conditions of a temperature of 20°C and a relative humidity of 65% by using Tensile Tester 3342 manufactured by Instron. A load cell capacity was set to 10 N, and a gripping tool was of clip type. The measurement value was set to an average value from the number of samples n = 5.

The toughness was determined according to the following calculation expression:

$$\text{Toughness} = [E / (r^2 \times \pi \times L) \times 1000] \text{ (unit: } MJ/m^3\text{)}$$

wherein

E: fracture energy (unit: J)
r: radius of the fiber (unit: mm)
π: ratio of the circumference of a circle
L: gripping distance at the time of tensile test measurement: 20 mm

(Comparative Example 6)

The preparation and evaluation of a sample were performed in the same way as in Example 4 except that xanthurenic acid was not used.

The rates of maintenance of elongation and toughness calculated according to the following expressions from elongation and toughness after 0.5 hours and after 4 hours from the start of UV irradiation are shown in Table 3 as to Example 4 and Comparative Example 6.

$$\text{Rate of maintenance of elongation (\%)} = \text{Elongation (\%) after 0.5 hours} / \text{Elongation (\%) after 4 hours} \times 100$$

$$\text{Rate of maintenance of toughness (\%)} = \text{Toughness } (MJ/m^3) \text{ after 0.5 hours} / \text{Toughness } (MJ/m^3) \text{ after 4 hours} \times 100$$

[Table 3]

| | Rate of maintenance of elongation (%) | Rate of maintenance of toughness (%) |
|---|---|---|
| Example 4 | 43.2 | 41.9 |
| Comparative Example 6 | 6.8 | 7.2 |

From Table 3, it is evident that the fiber comprising xanthurenic acid (Example 4), compared with the fiber comprising no xanthurenic acid (Comparative Example 6), had a high rate of maintenance as to both elongation and toughness and exhibited a UV resistance-improving effect.

## Claims

1. A microbial growth inhibitor comprising xanthurenic acid.

2. A method for inhibiting microbial growth in an article, the method comprising incorporating xanthurenic acid in the article.

3. The method according to claim 2, wherein the article comprises a protein.

4. The method according to claim 3, wherein the protein is a structural protein.

5. The method according to claim 4, wherein the structural protein comprises fibroin.

6. The method according to claim 5, wherein the fibroin is spider silk fibroin.

7. The method according to any one of claims 2 to 6, wherein the xanthurenic acid is incorporated at a ratio of 0.02 to 2.0% by mass with respect to a total mass of the article.

8. The method according to any one of claims 2 to 7, wherein the article is fiber.

9. A UV resistance improver comprising xanthurenic acid.

10. A method for improving UV resistance of an article, the method comprising incorporating xanthurenic acid in the article.

11. The method according to claim 10, wherein the article comprises a protein.

12. The method according to claim 11, wherein the protein is a structural protein.

13. The method according to claim 12, wherein the structural protein comprises fibroin.

14. The method according to claim 13, wherein the fibroin is spider silk fibroin.

15. The method according to any one of claims 12 to 14, wherein the xanthurenic acid is incorporated at a ratio of 0.02 to 2.0% by mass with respect to a total mass of the article.

16. The method according to any one of claims 12 to 15, wherein the article is fiber.

17. An artificial protein formed product comprising:

    an artificial protein; and
    xanthurenic acid.

18. The artificial protein formed product according to claim 17, wherein the artificial protein is a structural protein.

19. The artificial protein formed product according to claim 18, wherein the structural protein comprises fibroin.

**20.** The artificial protein formed product according to claim 19, wherein the fibroin is spider silk fibroin.

**21.** The artificial protein formed product according to any one of claims 17 to 20, wherein a content of the xanthurenic acid is 0.02 to 2.0% by mass with respect to a total mass of the artificial protein formed product.

**22.** The artificial protein formed product according to any one of claims 17 to 21, wherein the artificial protein formed product is fiber.

**23.** A method for manufacturing an artificial protein formed product, comprising:

preparing a solution comprising an artificial protein and xanthurenic acid; and
performing molding by using the solution.

**24.** The method for manufacturing an artificial protein formed product according to claim 23, wherein the artificial protein is a structural protein.

**25.** The method for manufacturing an artificial protein formed product according to claim 24, wherein the structural protein comprises fibroin.

**26.** The method for manufacturing an artificial protein formed product according to claim 25, wherein the fibroin is spider silk fibroin.

**27.** The method for manufacturing an artificial protein formed product according to any one of claims 24 to 26, wherein a content of the xanthurenic acid in the solution is 0.02 to 2.0% by mass with respect to a total mass of the solution.

**28.** An artificial protein solution comprising:

an artificial protein; and
xanthurenic acid.

**29.** The artificial protein solution according to claim 28, wherein the artificial protein is a structural protein.

**30.** The artificial protein solution according to claim 29, wherein the structural protein comprises fibroin.

**31.** The artificial protein solution according to claim 30, wherein the fibroin is spider silk fibroin.

**32.** The artificial protein solution according to any one of claims 28 to 31, wherein a content of the xanthurenic acid is 0.02 to 2.0% by mass with respect to a total mass of the artificial protein solution.

**33.** A coloring agent comprising xanthurenic acid.

*Fig.1*

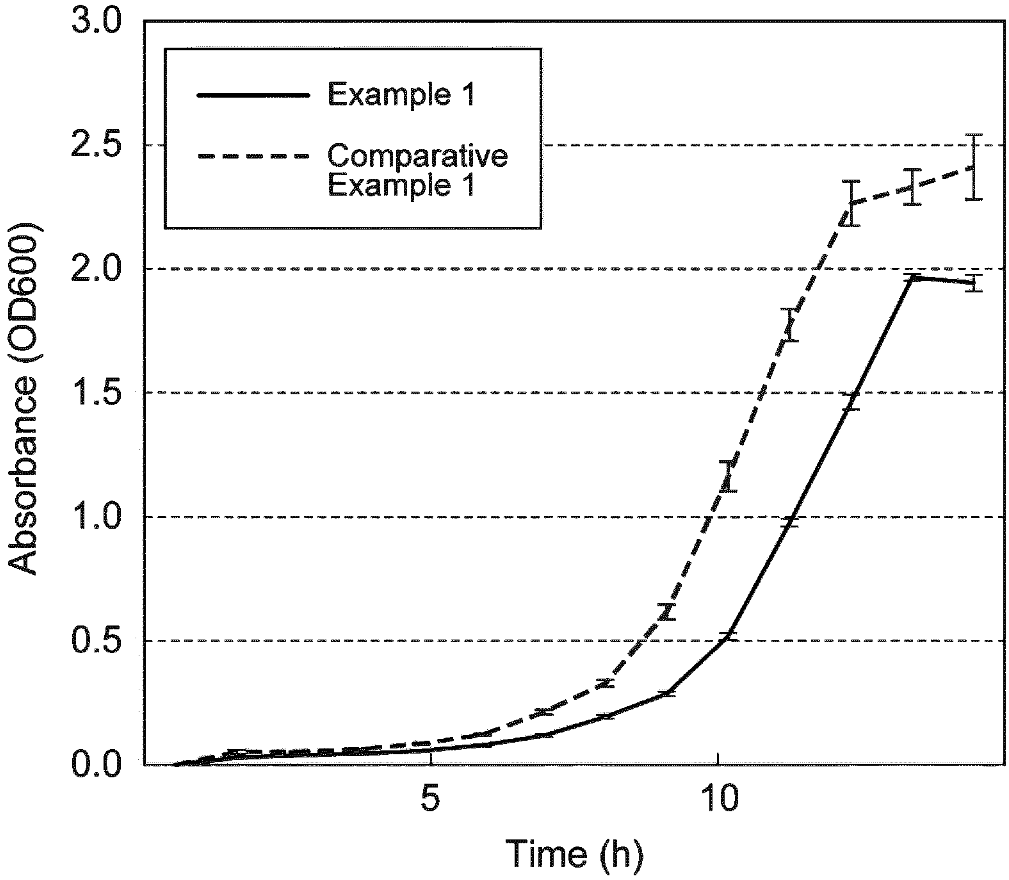
3.0
2.5
2.0
1.5
1.0
0.5
0.0
Absorbance (OD600)
5
10
Time (h)
Example 1
Comparative Example 1

*Fig.2*

(a)

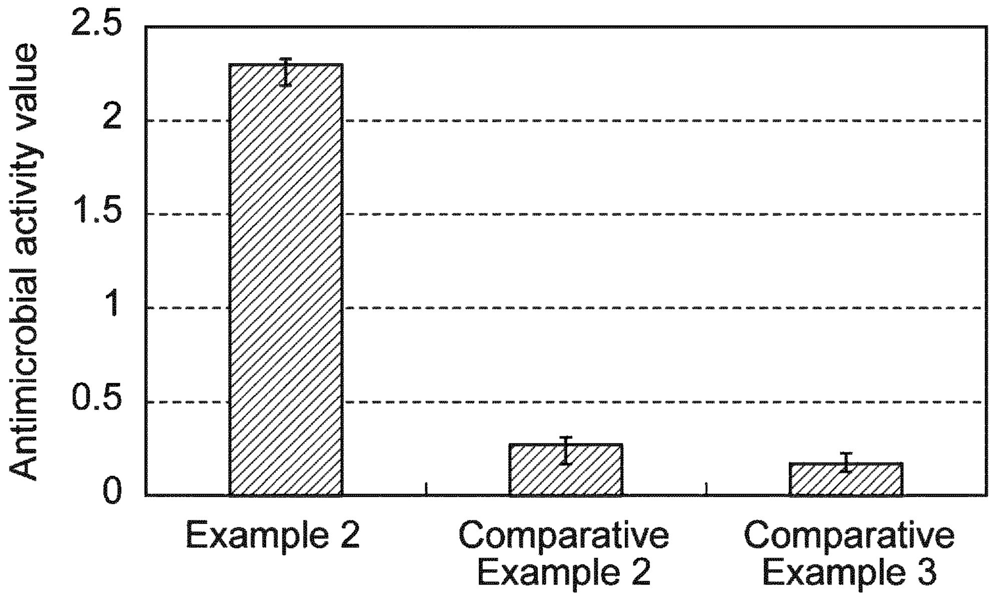
2.5
2
1.5
1
0.5
0
Antimicrobial activity value
Example 2
Comparative Example 2
Comparative Example 3

(b)

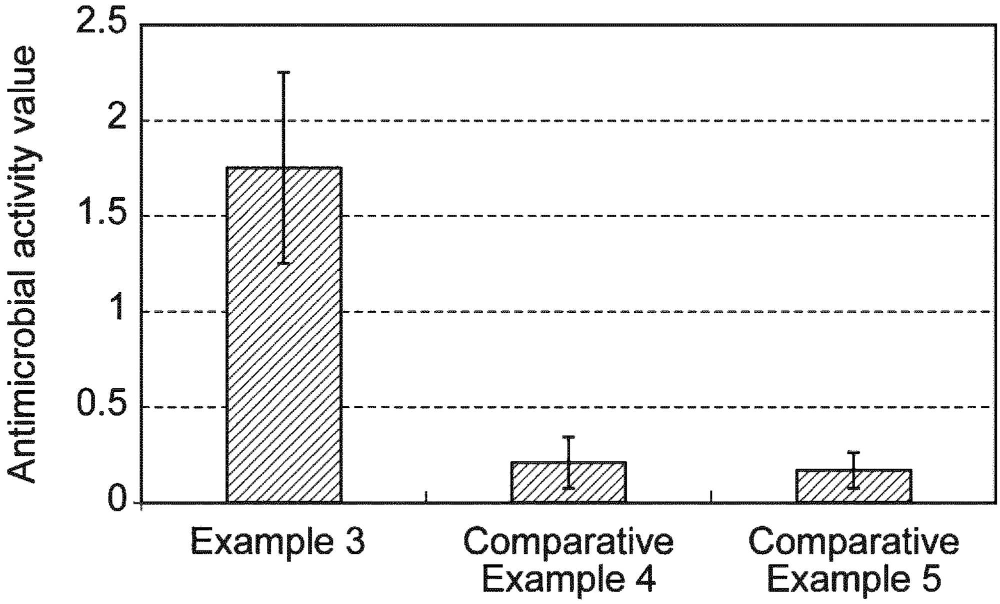
2.5
2
1.5
1
0.5
0
Antimicrobial activity value
Example 3
Comparative Example 4
Comparative Example 5

**INTERNATIONAL SEARCH REPORT**

International application No. PCT/JP2018/007561

A. CLASSIFICATION OF SUBJECT MATTER
Int.Cl. A01N43/42(2006.01)i, A01N25/10(2006.01)i, A01N25/34(2006.01)i, A01P3/00(2006.01)i, C07K14/78(2006.01)i

According to International Patent Classification (IPC) or to both national classification and IPC

B. FIELDS SEARCHED

Minimum documentation searched (classification system followed by classification symbols)
Int.Cl. A01N43/42, A01N25/10, A01N25/34, A01P3/00, C07K14/78

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY/MARPAT (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br><br>A | KAMAKSHI, V. Guru, et al., World Applied Sciences Journal, 2013, 25 (6), pp. 850-853, page 853, left column, line 29 to right column, line 3 | 1, 28<br>1-6, 8-14, 16-20, 22-26, 28-31, 33<br>7, 15, 21, 27, 32 |
| X<br>A | BANASIK, Marek, et al., The Journal of Biological Chemistry, 1992, 267 (3), pp. 1569-1575, page 1570, left column, lines 26-39 | 28, 32<br>1-27, 29-31, 33 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 April 2018 (05.04.2018) | 24 April 2018 (24.04.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/007561

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br><br>A | KOTAKE, Yashiro, et al., The Journal of Biochemistry, 1954, 41(4), pp. 425-433, EXPERIMENTAL | 33<br>1-6, 8-14, 16-20, 22-26, 28-31, 33<br>7, 15, 21, 27, 32 |
| Y<br><br>A | JP 2013-512265 A (AMSILK GMBH) 11 April 2013, claims, paragraphs [0299]-[0301] & US 2013/0109762 A1, claims, paragraphs [0360]-[0362] & WO 2011/063990 A2 & EP 2506837 A2 | 1-6, 8-14, 16-20, 22-26, 28-31, 33<br>7, 15, 21, 27, 32 |
| A | AZEREDO, L. da C., et al., Food Chemistry, 2003, 80, pp. 249-254, entire text | 1-33 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002114946 A
- JP 2016117862 A
- JP 2002238569 A


### Non-patent literature cited in the description


- *Proc. Natl. Acad. Sci. USA,* 1972, vol. 69, 2110
- Molecular Cloning